Europäisches Patentamt

European Patent Office

Office européen des brevets

(19)

(11) Numéro de publication: **0 266 287 B1**

# FASCICULE DE BREVET EUROPEEN

(45) Date de publication de fascicule du brevet:
16.10.91

(51) Int. Cl.5: **A01G 7/00, A01H 5/10**

(21) Numéro de dépôt: 87420290.6

(22) Date de dépôt: 28.10.87

(54) Procédé de régénération du tournesol par embryogénèse.

(30) Priorité: 30.10.86 FR 8615299

(43) Date de publication de la demande:
04.05.88 Bulletin 88/18

(45) Mention de la délivrance du brevet:
16.10.91 Bulletin 91/42

(84) Etats contractants désignés:
AT BE CH DE ES FR GB GR IT LI LU NL SE

(56) Documents cités:
EP-A- 0 160 390     EP-A- 0 170 904
EP-A- 0 171 593     EP-A- 0 172 377
FR-A- 2 547 981     US-A- 4 038 778
US-A- 4 354 327

(73) Titulaire: **RHONE-POULENC AGROCHIMIE**
**14-20, rue Pierre Baizet**
**F-69009 Lyon(FR)**

(72) Inventeur: **Freyssinet, Georges**
**21 rue de Nervieux**
**F-69450 St Cyr au Mont d'Or(FR)**
Inventeur: **Freyssinet, Martine**
**21 rue de Nervieux**
**F-69450 St Cyr au Mont d'Or(FR)**

(74) Mandataire: **Chrétien, François et al**
**RHONE-POULENC AGROCHIMIE- DPI B.P.**
**9163**
**F-69263 Lyon Cédex 09(FR)**

Rank Xerox (UK) Business Services

## Description

La présente invention a pour objet un procédé de régénération de plantes de tournesol, à partir de cellules ou de tissus, par embryogénèse somatique.

Bien que l'on puisse régénérer des plantes à partir de cellules isolées ou de tissus d'un grand nombre d'espèces cultivées, les efforts avec le tournesol ( Helianthus annuus) ont le plus souvent été vains. Quelques méthodes sont décrites dans la littérature, relatives à la régénération du tournesol par embryogénèse somatique, mais ces méthodes ne concernent que de rares génotypes, ou des hybrides interspécifiques. Des explants sont cultivés sur un premier milieu qui induit la formation d'un cal. Le cal est ensuite transféré sur un second milieu qui induit la formation d'embryons somatiques et de plantules. Puis les plantules sont transférées sur un troisième milieu qui induit la formation de racines et, à ce stade, les plantes régénérées peuvent être passées en terre.

PATERSON et EVERETT (Plant Sci., 1985, 42, 125) ont mis au point un milieu, optimisation de celui de GEORGIEVA-TODOROVA et al (Proc. 9th Int. Sunflower Conference, Torremolinos, Spain, 1980, 122) qui induit la régénération d'une espèce cultivée de tournesol, SS 415 B, part embryogénèse somatique à partir de fragments d'hypocotyles. Les explants sont cultivés à l'obscurité pendant 2 semaines, puis à la lumière pendant 2-4 semaines sur un milieu de Murashige et Skoog (MS) modifié, complémenté à 40 mg/l d'adénine sulfate, 1 mg/l de 6-benzylaminopurine (BAP), 1mg/l d'acide naphtalène acétique (ANA) et 0,1 mg/l de gibbéreline (GA₃). Pendant les 2 premières semaines il y a formation d'un cal friable, puis on voit apparaître à la lumière des taches vertes qui se transforment en pousses. Une étude anatomique révèle que ces taches vertes sont des embryons somatiques similaires aux embryons zygotiques.

CHANDLER et JAN (Agronomy Abstr., 1983, 58) décrivent la régénération du tournesol à partir d'embryons immatures issus de croisements interspécifiques. Les embryons immatures (4 à 5 jours) sont placés sur le milieu de croissance décrit par CHANDLER et BEARD pour le sauvetage d'embryons (Crop Sci., 1983, 23, 1004). Après 2 à 3 semaines, les embryons qui ont grossi sont transférés sur différentes formulations ayant pour base le milieu MS, avec des doses variables d'auxine et de cytokinine. La production de plantes sur ces milieux suit différentes voies, y compris la germination directe ou l'induction de cal suivie d'organogénèse ou d'embryogénèse. Les meilleurs résultats sont obtenus avec des concentrations d'acide indole acétique (AIA) et de kinétine entre 0 et 1 ppm. L'avantage de ce système est la production multiple de clones à partir d'un seul embryon.

COOLEY et WILCOX (demande de brevet européen 0172377) proposent un procédé appliqué à des cultivars de tournesol HA 89 et RHA 274 comprenant les trois étapes décrites ci-dessus et effectuées sur trois milieux de culture distincts, les milieux de culture des deux premières étapes contenant nécessairement une hormone de type auxinique. Cependant ce procédé présente plusieurs inconvénients, d'une part d'être applicable à des embryons de taille comprise dans une zone étroite (de 0,1 à 2 mm) donc avec peu de souplesse, d'autre part de nécessiter un changement de milieu entre la première et la seconde étape, enfin que le taux de régénération des plantules obtenu reste encore très insuffisant.

La présente invention concerne un procédé de régénération de cultivars de tournesol, plus simple et conduisant à des plantules de capacité améliorée de reprise en culture en terre.

Plus particulièrement, elle concerne un procédé de régénération de cultivars de tournesol selon la revendication 1.

Dans la suite de la description les pourcentages indiqués sont, sauf indication contraire en poids volume.

Les tissus végétaux d'où on peut tirer des explants pour la production d'embryons somatiques proviennent de cultivars de tournesol Helianthus annuus utilisés essentiellement dans les programmes de sélection. L'exemplification est faite avec huit cultivars :

HA 89, HA 290, HA 291, HA 300, HA 303, T 76, RT 26 et Giant Gray Stripe. La variété HA 89 provient de Dade Counts, Floride, les variétés HA 290, HA 291, HA 300, HA 303, T 76, et RT 26 de Seedtec International Inc., Woodland, Californie, et le Giant Gray Stripe de Northrup King Seeds, Fresno, Californie.

Les explants sont issus de plantes cultivées en serre et soumises à une fécondation contrôlée. L'explant préféré pour la production de cals est l'embryon immature. Les embryons immatures avec péricarpes sont isolés des capitules de tournesol lorsqu'ils sont agés de 4 à 21jours ; leur taille est généralement comprise entre 0,1 et 5 mm. Les embryons immatures de taille importante peuvent être coupés en plusieurs parties égales selon des plans passant par l'axe gemmule radicule, ce qui multiplie le nombre de clones obtenus à partir d'un seul embryon.

Le procédé selon l'invention est mis en oeuvre de la façon suivante :

Des variétés de tournesol HA 89 , HA 290, HA 291, HA 300, HA 303, T 76 , RT 26 et Giant Gray Stripe sont cultivées en serre (26°C, 40 000 lux, photopériode de 15 h par jour) et soumises à une fécondation contrôlée. Les embryons immatures sont prélevés 4 à 21 jours après pollinisation. Les graines détachées

2

des capitules sont stérilisées par immersion 20 minutes dans une solution d'eau de javel commerciale à 3° Cl additionnée de quelques gouttes d'un mouillant, puis lavées 3 fois à l'eau distillée stérile. Les embryons sont ensuite isolés en conditions d'asepsie, éventuellement coupés, et déposés sur le premier milieu dit d'induction des embryons.

Ce premier milieu comprend des sels minéraux, des vitamines, des acides aminés, du sucrose et une hormone en quantité suffisante pour la formation de cals. Les sels minéraux comprennent des sels de macro-éléments et des sels d'oligo-éléments. Les macro-éléments utilisés dans ce premier milieu peuvent être choisis par exemple parmi les composés suivants : sulfate de magnésium, chlorure de calcium, phosphate monopotassique, nitrate de potassium et nitrate d'ammonium. Parmi les sels à base d'oligo-éléments on peut citer : l'acide borique, le sulfate de manganèse, le sulfate de zinc, le molybdate de sodium, le sulfate cuivrique, le chlorure de cobalt, l'iodure de potassium, et le chélate de fer Na EDTA. Cette combinaison de sels minéraux est connue sous le nom de Murashige et Skoog (MS).

Les quantités préférées de macro-éléments et d'oligo-éléments pour un litre de milieu sont les suivantes :
- 370 mg de sulfate de magnésium heptahydraté,
- 440 mg de chlorure de calcium dihydraté,
- 170 mg de phosphate monopotassique dihydraté,
- 1900 mg de nitrate de potassium,
- 1650 mg de nitrate d'ammonium,
- 6,2 mg d'acide borique,
- 22,3 mg de sulfate de manganèse tétrahydraté,
- 8,6 mg de sulfate de zinc heptahydraté,
- 0,25 mg de molybdate de sodium dihydraté,
- 0,025 mg de sulfate cuivrique pentahydraté,
- 0,025 mg de chlorure de cobalt hexahydraté,
- 0,83 mg d'iodure de potassium,
- 36,7 mg de chélate de fer Na EDTA.

Le premier milieu contient aussi des vitamines telles que l'acide nicotinique, la thiamine, la pyridoxine, le myo-inositol et un acide aminé tel que alanine, glutamine, sérine, tryptophane, cystéine et de préférence la glycine. Les quantités préférées de vitamines et d'acide aminé pour un litre de milieu sont :
- 0,5 mg d'acide nicotinique,
- 0,1 mg de chlorhydrate de thiamine,
- 0,5 mg de chlorhydrate de pyridoxine,
- 100 mg de myo-inositol,
- 2 mg de glycine.

Le premier milieu peut également contenir un supplément de vitamine et d'acides aminés ; ce supplément n'est pas indispensable à la formation de embryons somatiques, mais il accroît leur fréquence. Dans cette éventualité, les quantités préférées de vitamines et d'acides aminés pour un litre de milieu seront :
- 0,5 mg d'acide nicotinique
- 0,1 mg de chlorydrate de thiamine
- 0,5 mg de chlorydrate de pyridoxine
- 4000 mg de myo-inositol
- 1000 mg de L-alanine
- 800 mg de L-glutamine
- 160 mg de L'sérine
- 50 mg de L-tryptophane
- 10 mg de L-cystéine
- 2 mg de glycine

Le sucrose contenu dans le premier milieu est présent à raison de 8 à 12 %, de préférence 9 %.

L'hormone du premier milieu, dont le choix est une caractéristique de l'invention, est de type cytokinine, par exemple la kinétine ou la 6-benzylaminopurine, et de préférence cette dernière. Généralement, des quantités de 0,5 à 1 mg par litre de milieu conviennent. Une gélose telle que le Phytagar ou le Gelrite est utilisée pour rendre le milieu solide. Une concentration finale de 0,6 % pour le Phytagar ou de 0,3 % pour le Gelrite donne de bons résultats. Le pH du milieu peut varier de 5,0 à 6,3 et est de préférence 5,0.

Le milieu est stérilisé en autoclave à l'exception des hormones qui sont stérilisées par filtration sur membrane microporeuse.

Les embryons immatures, isolés comme décrit ci-dessus, déposés sur ce premier milieu, sont cultivés pendant environ 2 à 3 semaines à l'obscurité, à 26° C. Pendant cette période les embryons zygotiques se développent et des embryons somatiques apparaissent au niveau de l'hypocotyle et sur la face interne des cotylédons dans le cas du cultivar HA 291 . Ensuite, les embryons somatiques peuvent être isolés et déposés sur le deuxième milieu dit milieu de croissance des plantules. Le cal embryogène ou les embryons isolés sont cultivés sur le second milieu pendant 2 à 3 semaines à 26° C à la lumière (1500 lux) avec une photopériode de 12 à 16 h par jour, de préférence 16 h par jour. Pendant cette période, les embryons germent et les plantules formées développent éventuellement des racines.

Le deuxième milieu contient, comme le premier milieu, des sels minéraux, des vitamines, un acide aminé, du sucrose et une hormone. Les sels minéraux comprennent des sels de macro-éléments et des sels d'oligo-éléments. Les macro-éléments, les oligo-éléments, les vitamines et l'acide aminé sont les mêmes que dans le premier milieu, en concentrations identiques sans complément. Le sucrose est présent en quantité de préférence inférieure à celle du premier milieu par exemple de 2 à 4 %, de préférence 3 %. L'hormone utilisée est une cytokinine, de préférence identique à (mais pouvant être différente de) celle du premier milieu, de préférence la 6-benzylaminopurine. Généralement des quantités de 0,1 à 0,5 mg par litre conviennent. Une gélose telle que Phytagar ou le Gelrite est utilisée pour rendre le milieu solide. Une concentration finale de 0,6 % pour le Phytagar et de 0,3 % pour le Gelrite donne de bons résultats. Le milieu est stérilisé comme précédemment, et a un pH de 5,0 à 6,3, avec une préférence pour un pH de 5,0.

Après 2 à 3 semaines sur le second milieu, les embryons somatiques isolés ont germé et se sont transformés en plantules de 2 à 5 cm. Les plantules qui ont développé des racines peuvent être passées en terre, dans un mélange stérile de tourbe, vermiculite, sable fin (3:2:1) contenu dans des Jiffy-pots ; les pots sont placés dans un bac rempli de sable humide et recouverts d'un sac de polyéthylène transparent pour maintenir un haut degré d'humidité. L'ensemble est placé dans une chambre climatique à 15° C (6000 lux), avec une photopériode de 12 à 16 h par jour, de préférence 15 h par jour. Après 10 jours, les plantes sont passées dans des pots plus grands et cultivées en serre à 26° C (40 000 lux), avec une photopériode de 12 à 16 h par jour, de préférence 15 h par jour.

Les plantes qui n'ont pas développé de racines peuvent être soit greffées sur des jeunes plantes cultivées en serre selon une méthode classique (cf., par exemple, HABERMANN et WALLACE, Amer. J. Bot., 1958, 45, 479) soit transférées pour une période de 10 à 20 jours sur un milieu d'enracinement, ou troisième milieu.

Le troisième milieu comprend des sels minéraux, des vitamines et du sucrose. Les sels minéraux comprennent des sels de macro-éléments et des sels d'oligo-éléments. Les macro-éléments utilisés dans ce troisième milieu peuvent être choisis parmi les composés suivants : sulfate de magnésium, chlorure de calcium, phosphate monosodique, nitrate de potassium, et sulfate d'ammonium. Parmi les sels à base d'oligo-éléments, on peut citer l'acide borique, le sulfate de manganèse, le sulfate de zinc, le molybdate de sodium, le sulfate cuivrique, le chlorure de cobalt, l'iodure de potassium et le chélate de fer Na EDTA. Cette combinaison de sels minéraux est connue sous le nom de B5. Les quantités préférées de macro-éléments et d'oligo-éléments pour un litre de milieu sont les suivantes :
- 250 mg de sulfate de magnésium heptahydraté,
- 150 mg de chlorure de calcium dihydraté,
- 169 mg de phosphate monosodique monohydraté,
- 3 000 mg de nitrate de potassium,
- 3 mg d'acide borique,
- 13,2 mg de sulfate de manganèse tétrahydraté,
- 2 mg de sulfate de zinc heptahydraté,
- 0,25 mg de molybdate de sodium dihydraté,
- 0,025 mg de sulfate cuivrique pentahydraté,
- 0,025 mg de chlorure de cobalt hexahydraté,
- 0,75 mg d'iodure de potassium,
- 40 mg de chélate de fer Na EDTA.

Le troisième milieu contient aussi des vitamines. Ce sont l'acide nicotinique, la thiamine, la pyridoxine et le myo-inositol. Les quantités préférées de vitamines sont, pour un litre de milieu :
- 1 mg d'acide nicotinique,
- 10 mg de chlorhydrate de thiamine,
- 1 mg de chlorhydrate de pyridoxine et,
- 100 mg de myo-inositol.

Le troisième milieu contient également du sucrose et du charbon activé/ou une hormone de type auxinique. Le sucrose est présent par exemple à raison de 1 à 2 %, de préférence 1 %, et le charbon

EP 0 266 287 B1

activé à raison de 0,1 %. Si l'on préfère utiliser une hormone à la place du charbon activé, on choisira l'acide indole-3-acétique, à des doses de 0,1 à 0,2 mg par litre. Une gélose telle que le Phytagar ou le Gelrite est utilisée pour rendre le milieu solide. Une concentration finale de 0,5 % pour le Phytagar et de 0,25 % pour le Gelrite est suffisante. Le pH du milieu peut varier de 5,0 à 6,0 et est de préférence 5,0. Le milieu est stérilisé en autoclave.

Les plantes sont cultivées sur ce milieu pendant 2 à 3 semaines à 26°C à la lumière (1500 lux), avec une photopériode de 12 à 16 h par jour, et de préférence 16 h par jour. Les plantes forment alors des racines, et peuvent être passées en terre.

Les plantes obtenues par ce procédé peuvent être phénotypiquement différentes du matériel de départ, du fait du stress in vitro. Les plantes sont pollinisées à la main et conservées jusqu'à la récolte des graines. Quelques unes des graines sont plantées pour l'analyse des nouveaux plants de tournesol et la sélection d'éventuels mutants intéressants qui sont alors intégrés dans un programme de sélection variétale.

La présente invention sera décrite de manière plus complète mais non limitative dans les exemples ci-dessous :

Exemple I - Préparation des solutions mères

a) Solution mère de B5

Une solution mère de B5, 10 fois concentrée, est préparée en dissolvant un sachet de milieu B5 sans sucrose de Flow Laboratories (contient les sels minéraux du B5, 1 mg d'acide nicotinique, 1 mg de pyridoxine HCl, 10 mg de thiamine HCl et 100 mg d'inositol, concentrations finales) dans 1 000 ml (volume final) d'eau distillée et déionisée. La solution mère est divisée en aliquotes de 100 ml et conservée à -20°C.

b) Solution mère de BAP

La solution de BAP à 1 g/l est préparée en dissolvant 100 mg de 6-benzylaminopurine dans quelques ml d'HCl 0.15 N, en chauffant légèrement, et en diluant à 100 ml avec de l'eau distillée et déionisée. Cette solution est stérilisée par filtration sur une membrane Minisart NML 0,2 micron (Sartorius) avant son addition aux premier et deuxième mileux.

c) Solution mère d'AIA

La solution d'AIA à 0,2 g/l est préparée en dissolvant 20 mg d'acide indole-3-acétique dans quelques ml d'éthanol pur, et en diluant à 100 ml avec de l'eau distillée et déionisée. Cette solution est stérilisée par filtration sur une membrane Minisart NML 0,2 micron (Sartorious) avant son addition au troisième milieu.

d) Supplément de vitamine et d'acides aminés
selon Chandler et BEARD (Crop. Sci., 1983, 23, 1004)

Une solution mère de vitamine et d'acides aminés, 20 fois concentrée, est préparée en dissolvant ensemble 39 g de myo-inositol, 10 g de L'alanine, 8 g de L-glutamine, 1,6 g de L-sérine, 0,5 g de L-tryptophane et 0,1 g de L-cystéine dans 500 ml (volume final) d'eau distillée et déionisée. La solution mère est divisée en parties aliquotes de 50 ml et conservée à -20°C.

Example II - Préparation des milieux

a) Premier milieu ou milieu d'induction des embryons

Il est préparé par dissolution d'un sachet de milieu de Murashige et Skoog sans sucrose de Flow Laboratories (contient les sels minéraux de MS, 0,1 mg de thiamine HCl, 0,5 mg d'acide nicotinique, 0,5 mg de pyridoxine HCl, 2 mg de glycine et 100 mg d'inositol) et 90 à 120 g de sucrose dans de l'eau distillée et déionisée. Après éventuelle addition de 50 ml du supplément de vitamine et d'acides aminés, le pH est ajusté à 5,0 avec de la soude 0,1 N, le volume amené à 1 litre, et après addition de 6 g de Phytagar (Gibco) ou 3 g de Gelrite (Kelco), le mélange est autoclavé 20 minutes à 120 psi. Quand le milieu est tiède on y ajoute 0,5 à 1 ml de la solution de BAP (2,2 - 4,4 micromole) stérilisée comme décrit plus haut. Le mélange est ensuite coulé en boites de Pétri.

5

b) Deuxième milieu ou milieu de croissance des plantules.

Le deuxième milieu est préparé de façon identique au premier milieu, en dissolvant ensemble un sachet de milieu MS, 30 g de sucrose et 6 g de Phytagar ou 3 g de Gelrite dans un litre d'eau, et en ajoutant, après passage à l'autoclave 0,1 à 0,5 ml de la solution de BAP stérile (0,44 - 2,2 micromole). Le milieu est coulé dans des boites Plantcon (Flow Laboratories).

c) Troisième milieu ou milieu d'enracinement

Le troisième milieu est préparé en ajoutant 10 à 20/g de sucrose à 100 ml de la solution mère de B5, dans de l'eau distillée et déionisée. Le pH est ajusté à 5,0 par de la soude 0,1 N, puis le volume est amené à 1 litre et on ajoute au mélange 5 g de Phytagar et 1 g de charbon activé. Après passage à l'autoclave le milieu est coulé dans des Mason jars. Si on désire utiliser un troisième milieu contenant de l'AIA, le charbon activé est omis lors de la préparation de ce troisième milieu. Après passage à l'autoclave, on ajoute dans le milieu tiède 0,5 à 1 ml de la solution d'AIA (0,57 -1,14 micromole) stérile. Le milieu est coulé de façon identique dans des Mason jars.

Une variante liquide du troisième milieu comprend uniquement les sels minéraux du B5 et 1 % de sucrose, à pH 5,0. Le mélange est distribué par fractions de 10 ml dans des tubes de verre contenant des ponts de papier filtre, selon la méthode décrite par CHANDLER et BEARD (Crop Sci., 1983, 23, 1004), puis passage à l'autoclave.

Exemple III

Les embryons immatures avec leurs péricarpes sont séparés du capitule de tournesol ( Helianthus annuus, var. T 76 B) quand ils atteignent 0,5 à 1,5 mm. Les graines sont stérilisées 20 minutes dans une solution d'eau de javel à 3 degrés chlorométriques , et rincées à l'eau distillée. Les embryons immatures sont séparés de leurs enveloppes et déposés en boites de Pétri sur le premier milieu. Le premier milieu est préparé de la façon décrite ci-dessus, avec 90 g/l de sucrose et 0,5 mg/l de BAP (2,2 micromole).

Les boites de Pétri sont incubées à l'obscurité pendant 3 semaines pour induire la formation d'embryons somatiques.

A ce stade, les embryons somatiques qui ont commencé à germer sont séparés de l'embryon zygotique et déposés sur le second milieu, dans des boites Plantcon. le second milieu, préparé comme décrit plus haut, contient 0,1 mg de BAP par litre, soit 0,44 micromole. Les embryons sont cultivés à la lumière pendant 3 semaines, et se différencient en plantules.

Les plantules de 2 à 5 cm de hauteur sont ensuite transférées sur le troisième milieu pour initier la formation des racines. Le troisième milieu, préparé comme décrit précédemment contient de préférence 10 g de sucrose. Dans le cas du milieu gélifié, l'addition de 1 g/l de charbon activé élimine l'influence résiduaire du BAP fourni par le milieu précédent (MAENE et DEBERGH, PLant Cell Tissue Organ Culture, 1985, 5 23-33) et favorise une croissance rapide des racines. Après environ 2 semaines, les plantules peuvent être passées en terre.

Si les plantules ont commencé à développer des racines sur le deuxième milieu, elles peuvent être mises en contact du troisième milieu liquide, selon la technique décrite par CHANDLER et BEARD (Crop Sci. 1983, 23, 1004). Ce dispositif permet un développement important du système racinaire, et évite de blesser les racines lors du passage en terre.

Exemple IV

Les embryons immatures de Helianthus annuus, var. HA 89, sont prélevés quand ils atteignent 0,1 à 0,5 mm et déposés, avec l'endosperme, sur le premier milieu. Le premier milieu contient 90 g de sucrose et 0,5 mg de BAP par litre, soit 2,2 micromole. Après 3 semaines à l'obscurité, les embryons somatiques sont isolés et déposés sur du premier milieu frais, toujours à l'obscurité. Des embryons somatiques secondaires vont se former sur les embryons isolés. Après 3 semaines à l'obscurité, les embryons somatiques secondaires sont isolés et déposés sur le deuxième milieu. Le deuxième milieu contient 30 g de sucrose et 0,1 mg de BAP par litre (0,44 micromole).

Les plantules qui vont se développer pourront ensuite être transférées sur le troisième milieu, liquide ou gélifié, pour s'enraciner. Elles seront ensuite passées en terre.

Exemple V

Les embryons immatures de Helianthus annuus, var. HA 89, sont prélevés 21 jours après pollinisation ; ils ont une taille de 5 mm. Ils sont séparés de leurs enveloppes et, avant d'être déposés sur le premier milieu, ils sont coupés en quatre parties égales, longitudinalement, selon deux plans perpendiculaires entre eux et parallèles à l'axe gemmule-radicule. Dans ce cas, le premier milieu contient 90 g de sucrose et 1 mg de BAP par litre. Après 3 semaines à l'obscurité, les embryons somatiques sont isolés et déposés sur le deuxième milieu. Le deuxième milieu contient 30 g de sucrose et 0,5 mg de BAP par litre.

Les plantules qui vont se développer pourront ensuite être transférées sur le troisième milieu ( liquide on gélifié) pour s'enraciner, puis passées en terre. Les plantules qui n'ont pas fait de racines peuvent être greffées sur une jeune plante, selon la méthode décrite par HABERMANN et WALLACE (Amer. J. Bot., 1958, 45, 479).

Exemples 6-27

Les exemples ci-dessous donnent une idée non exhaustive des combinaisons que l'on peut effectuer en utilisant les différentes modalités décrites.

| Exemple | Variété | Taille (mm) | Sections | Milieu I | | | Milieu II | | | Enracinement |
|---|---|---|---|---|---|---|---|---|---|---|
| | | | | Milieu de base | Sucrose % | BAP mg/l | Milieu de base | Sucrose % | BAP mg/l | |
| 6 | T 76 | 1,5 | – | MS mod* | 9 | 1 | MS mod* | 3 | 1 | MS mod* +3% su-crose + 0,5 BAP |
| 7 | T 76 | 1,5 | – | MS mod* | 9 | 1 | MS mod* | 3 | 1 | III SC |
| 8 | T 76 | 1,5 | – | MS mod* | 9 | 1 | MS mod* | 3 | 1 | III L |
| 9 | T 76 | 1,5 | – | MS | 9 | 1 | MS | 3 | 1 | III SC |
| 10 | T 76 | 1,5 | – | MS | 12 | 0,5 | MS | 3 | 0,1 | III SC |
| 11 | T 76 | 1,5 | – | MS | 12 | 0,5 | MS | 3 | 0,1 | III L |
| 12 | T 76 | 1,5 | – | MS sup ** | 9 | 0,5 | MS | 3 | 0,1 | III SA 0,1 |
| 13 | T 76 | ≤3 | – | MS | 9 | 0,5 | MS | 3 | 0,5 | III SC |
| 14 | T 76 | ≤3 | – | MS | 9 | 0,5 | MS | 3 | 0,5 | III L |
| 15 | T 76 | = 5 | + | MS | 9 | 0,5 | MS | 3 | 0,5 | IIISC |
| 16 | T 76 | = 5 | + | MS | 9 | 0,5 | MS | 3 | 0,5 | III L |
| 17 | RT 26 | ≤1,5 | – | MS | 12 | 0,5 | MS | 3 | 0,1 | III L |
| 18 | RT 26 | ≤3 | – | MS sup ** | 9 | 0,5 | MS | 3 | 0,1 | III SC |
| 19 | HA 89 | ≤1,5 | – | MS | 12 | 0,5 | MS | 3 | 0,5 | III SC |
| 20 | HA 89 | ≤3 | – | MS | 9 | 0,5 | MS | 3 | 0,5 | III SC |
| 21 | HA 89 | ≤3 | – | MS | 9 | 0,5 | MS | 3 | 0,5 | III L |
| 22 | HA 89 | ≤3 | – | MS sup ** | 9 | 0,5 | MS | 3 | 0,1 | III SC |
| 23 | HA 89 | = 5 | + | MS | 9 | 0,5 | MS | 3 | 0,5 | III SC |
| 24 | HA 89 | = 5 | + | MS | 9 | 0,5 | MS | 3 | 0,5 | III L |
| 25 | HA 89 | >5 | + | MS | 9 | 1 | MS | 3 | 0,5 | III SC |
| 26 | HA 291 | ≤1,5 | – | MS sup ** | 9 | 0,5 | MS | 3 | 0,1 | III SA 0,1 |
| 27 | HA 291 | = 5 | + | MS sup ** | 9 | 0,5 | MS | 3 | 0,1 | III SA 0,2 |

* Le milieu MS modifié contient 600 mg de chlorure de calcium dihydraté, 1260 mg de nitrate de potassium, 1100 mg de nitrate d'ammonium et 250 mg de myo-inositol par litre de milieu. Les autres éléments restent inchangés.

** milieu MS supplémenté

III SA = milieu III solide + AIA (0,1 ou 0,2 mg/l)

8

III SC = milieu III solide + charbon activé

III L = milieu III liquide

## Revendications

1. Procédé de régénération de cultivars de tournesol Helianthus annuus par embryogénèse somatique à partir de cellules ou de tissus comprenant successivement :
   - une étape de culture de tissus de tournesol dans un premier milieu nutritif d'induction contenant une hormone pour la formation de cals embyogènes et d'embryons somatiques, et la croissance desdits embryons :
   - une seconde étape consistant en une culture permettant la germination desdits embryons et leur développement en plantes éventuellement pourvues de racines, sur un second milieu dit de croissance des plantules ;
   - éventuellement une troisième étape pour la croissance ou la formation des racines desdites plantes sur un milieu dit d'enracinement ;
   
   caractérisé en ce que les deux premières étapes sont effectuées sur un milieu de même nature, que ce milieu contient une quantité efficace d'une hormone de type cytokinine, sans hormone auxinique, et que le premier milieu contient de 8 à 12 % de sucrose et le second milieu de 2 à 4 % de sucrose.

2. Procédé selon la revendication 1, caractérisé en ce que lesdits tissus sont des embryons immatures.

3. Procédé selon l'une des revendications 1 et 2, caractérisé en ce que l'hormone est choisie dans le groupe comprenant la kinétine, la 6-benzyl aminopurine, la 2-isopentyladénine et la zéatine.

4. Procédé selon la revendication 3, caractérisé en ce que l'hormone est la 6-benzylaminopurine à des concentrations de 0,1 à 8 micromole dans les 1er et 2ème milieux.

5. Procédé selon l'une des revendications 1 à 4, caractérisé en ce que les concentrations de 6-benzylaminopurine sont de 2,2 - 4,4 micromole dans le premier milieu, et de 0,44 - 4,4 micromole dans le deuxième milieu.

6. Procédé selon l'une des revendications 1 à 5, caractérisé en ce que les concentrations en sucrose sont de 9 % dans le premier milieu et de 3 % dans le deuxième milieu.

7. Procédé selon l'une des revendications 1 à 6, caractérisé en ce qu'on utilise comme premier milieu un milieu de type Murashige et Skoog (MS).

8. Procédé selon l'une des revendications 1 à 6, caractérisé en ce qu'on utilise comme premier milieu un milieu de type Murashige et Skoog (MS) contenant un supplément en vitamine et acides aminés.

9. Procédé selon l'une des revendications 1 à 8, caractérisé en ce que le second milieu contient des sels minéraux, des vitamines et un acide aminé en quantités identiques à celles du premier milieu non supplémenté en vitamine et en acides aminés.

10. Procédé selon l'une des revendications 1 à 9, caractérisé en ce que l'on met en culture des embryons immatures âgés de 4 à 21 jours, et de taille comprise entre 0,1 et 5 mm, et que lesdits embryons peuvent être découpés en plusieurs fragments régénérables.

11. Procédé selon l'une des revendications 1 à 10, caractérisé en ce qu'on utilise les cultivars T 76, RT 26, HA 89, HA 290, HA 291, HA 300, HA 303 ou Giant Gray Stripe.

12. Procédé selon les revendications 1 et 11, caractérisé en ce que, après la seconde étape, les plantes obtenues sont mises en culture d'induction racinaire.

13. Procédé selon l'une des revendications 1 à 11, caractérisé en ce que, après la seconde étape, les plantes porteuses de racines sont passées en terre et incubées dans une chambre climatique à 15° C, avec une intensité lumineuse de 6000 lux et une photopériode de 15 h par jour.

**14.** Procédé selon l'une des revendications 1 à 11, caractérisé en ce que, après la seconde étape, les plantes obtenues sont greffées.

**Claims**

1. A process for regenerating cultivars of sunflower Helianthus annuus by somatic embryogenesis from cells or tissues, comprising successively:
   - a stage of culturing sunflower tissues in a first nutrient induction medium containing a hormone, for the formation of embryogenic calluses and somatic embryos, and the growth of the said embryos;
   - a second stage consisting of a culturing which permits the germination of the said embryos and their development to plants, possibly provided with roots, on a second so-called plantlet growth medium;
   - where appropriate, a third stage for the growth or formation of the roots of the said plants on a so-called rooting medium;
   
   wherein the first two stages are performed on a medium of the same nature, wherein this medium contains an effective quantity of a hormone of the cytokinin type, without an auxin type hormone, and wherein the first medium contains from 8 to 12 % of sucrose and the second medium from 2 to 4 % of sucrose.

2. The process according to claim 1, wherein the said tissues are immature embryos.

3. The process according to one of claims 1 and 2, wherein the hormone is chosen from the group comprising kinetin, 6-benzylaminopurine, 2-isopentyladenine and zeatin.

4. The process according to claim 3, wherein the hormone is 6-benzylaminopurine at concentrations of 0.1 to 8 micromolar in the 1st and 2nd media.

5. The process according to one of claims 1 to 4, wherein the concentrations of 6-benzylaminopurine are 2.2 - 4.4 micromolar in the first medium and 0.44 - 4.4 micromolar in the second medium.

6. The process according to one of claims 1 to 5, wherein the concentrations of sucrose are 9% in the first medium and 3% in the second medium.

7. The process according to one of claims 1 to 6, wherein a medium of the Murashige and Skoog (MS) type is used as the first medium.

8. The process according to one of claims 1 to 6, wherein a medium of the Murashige and Skoog (MS) type containing a vitamin and amino acid supplement is used as the first medium.

9. The process according to one of claims 1 to 8, wherein the second medium contains inorganic salts, vitamins and an amino acid, in quantities identical to those in the first medium which is not supplemented with vitamin and amino acids.

10. The process according to one of claims 1 to 9, wherein immature embryos from 4 to 21 days old and between 0.1 and 5 mm in size are cultured, and wherein the said embryos can be cut up into several fragments capable of regeneration.

11. The process according to one of claims 1 to 10, wherein the cultivars T 76, RT 26, HA 89, HA 290, HA 291, HA 300, HA 303 or Giant Gray Stripe are used.

12. The process according to claims 1 and 11, wherein, after the second stage, the plants obtained are cultured for root induction.

13. The process according to one of claims 1 to 11, wherein, after the second stage, the plants bearing roots are moved to soil and incubated in a climatic chamber at 15° C with a light intensity of 6,000 lux and a photo-period of 15 h per day.

**14.** The process according to one of claims 1 to 11, wherein, after the second stage, the plants obtained are grafted.

## Patentansprüche

**1.** Verfahren zur Regeneration von Sonnenblumencultivars (Helianthus annuus) durch somatische Embryogenese aus Zellen oder Geweben, welches Verfahren aufeinanderfolgend umfaßt:
- eine Stufe der Züchtung voll Sonnenblumengeweben in einem ersten Nährmedium, das ein Hormon zur Bildung von embryogenem Kallus und somatischen Embryonen enthält, zwecks Induktion und Wachstum der Embryonen;
- eine zweite Züchtungsstufe, die die Keimung der Embryonen und ihre Entwicklung zu gegebenenfalls mit Wurzeln versehenen Pflanzen ermöglicht, auf einem zweiten Milieu für das Keimlingswachstum;
- gegebenenfalls eine dritte Stufe zwecks Wachstums oder Wurzelbildung der Pflanzen auf einem Bewurzelungsmilieu;
dadurch gekennzeichnet, daß die beiden ersten Stufen auf einem gleichartigen Milieu ausgeführt werden, daß dieses Milieu eine wirksame Menge eines Hormons vom Cytokinintyp ohne Auxinhormon enthält und daß das erste Milieu 8 bis 12 % Saccharose und das zweite Milieu 2 bis 4 % Saccharose enthält.

**2.** Verfahren nach Anspruch 1, dadurch gekennzeichnet, daß die Gewebe unreife Embryonen sind.

**3.** Verfahren nach einem der Ansprüche 1 und 2, dadurch gekennzeichnet, daß das Hormon ausgewählt ist aus der Gruppe, umfassend Kinetin, 6-Benzylaminopurin, 2-Isopentyladenin und Zeatin.

**4.** Verfahren nach Anspruch 3, dadurch gekennzeichnet, daß das Hormon 6-Benzylaminopurin in Konzentrationen von 0,1 bis 8 mikromol im ersten und zweiten Milieu ist.

**5.** Verfahren nach einen, der Ansprüche 1 bis 4, dadurch gekennzeichnet, daß die Konzentration von 6-Benzylaminopurin im ersten Milieu 2,2 bis 4,4 mikromol und im zweiten Milieu 0,44 bis 4,4 mikromol ist.

**6.** Verfahren nach einem der Ansprüche 1 bis 5, dadurch gekennzeichnet, daß die Saccharosekonzentration 9% im ersten Milieu und 3 % im zweiten Milieu ist.

**7.** Verfahren nach einem der Ansprüche 1 bis 6, dadurch gekennzeichnet, daß man als erstes Milieu ein Medium vom Typ Murashige und Skoog (MS) verwendet.

**8.** Verfahren nach einem der Ansprüche 1 bis 6, dadurch gekennzeichnet, daß man als erstes Milieu ein Medium vom Typ Murashige und Skoog (MS) verwendet, das ein Vitamin- und Aminosäure-Supplement enthält.

**9.** Verfahren nach einem der Ansprüche 1 bis 8, dadurch gekennzeichnet, daß das zweite Milieu anorganische Salze, Vitamine und eine Aminosäure in gleichen Mengen wie das erste Milieu ohne Vitamin- und Aminosäure-Supplement enthält.

**10.** Verfahren nach einem der Ansprüche 1 bis 9, dadurch gekennzeichnet, daß man unreife Embryonen im Alter von 4 bis 21 Tagen und einer Größe zwischen 0,1 und 5 mm züchtet, und daß die Embryonen in mehrere regenerierbare Fragmente geschnitten sein können.

**11.** Verfahren nach einem der Ansprüche 1 bis 10, dadurch gekennzeichnet, daß man die Cultivars T 76, RT 26, HA 89, HA 290, HA 291, HA 300, HA 303 oder Giant Gray Stripe verwendet.

**12.** Verfahren nach den Ansprüchen 1 und 11, dadurch gekennzeichnet, daß die nach der zweiten Stufe erhaltenen Pflanzen in eine Kultur zur Induktion der Wurzelbildung gesetzt werden.

**13.** Verfahren nach einem der Ansprüche 1 bis 11, dadurch gekennzeichnet, daß die Wurzeln tragenden Pflanzen nach der zweiten Stufe in Erde gesetzt und in einer Klimakammer bei 15°C mit einer Lichtintensität von 6000 Lux und einer Photoperiode von 15 h pro Tag inkubiert werden.

14. Verfahren nach einem der Ansprüche 1 bis 11, dadurch gekennzeichnet, daß die nach der zweiten Stufe erhaltenen Pflanzen gepfropft werden.